# EUROPEAN PATENT APPLICATION

(11) **EP 3 015 170 A1**
(43) Date of publication of application: **04.05.2016**
(21) Application number: 15191846.3
(22) Date of filing: 28.10.2015
(51) Int. Cl.: B01L 7/00, C12Q 1/68

(54) **METHOD FOR DISCRIMINATING PRESENCE OR ABSENCE OF MUTATION IN DNA**

(30) Priority: 30.10.2014 JP 2014221817; 19.08.2015 JP 2015161672
(71) Applicant: Seiko Epson Corporation, Shinjuku-ku Tokyo 163-0811 (JP)
(72) Inventor: Uehara, Masayuki, Nagano 390-8621 (JP); Idegami, Kotaro, Nagano 392-8502 (JP)
(74) Representative: Hoffmann Eitle

(57) **Abstract**

A method for discriminating the presence or absence of a mutation having a plurality of mutated forms in a DNA includes performing a thermal cycle for amplifying the DNA in the presence of a pair of primers which can bind to the DNA, a first probe which does not bind to the DNA having a mutation, can bind to the DNA having no mutation, and is labeled with a first fluorescent substance, and a second probe which can bind to both of the DNA having a mutation and the DNA having no mutation and is labeled with a second fluorescent substance which emits a light having a fluorescence wavelength different from that of a light emitted from the first fluorescent substance.

SEQUENCE LISTING
<110> Seiko Epson Corporation

<120> Method for discriminating presence or absense of mutation in DNA

<130> J018274201

<160> 4

<150> JP 2014-221817
<151> 2014-10-30

<150> JP 2015-161672
<151> 2015-08-19

<170> PatentIn version 3.5

<210> 1
<211> 22
<212> DNA
<213> Artificial Sequence

<220>
<223> PCR forward primer

<400> 1
atccaggtac gggtgaagac ac                                                22

<210> 2
<211> 23
<212> DNA
<213> Artificial Sequence

<220>
<223> PCR reverse primer

<400> 2
cgcatcaaca agtcctagcg aac                                               23

<210> 3
<211> 15
<212> DNA
<213> Artificial Sequence

<220>
<223> PCR probe

<400> 3
cgggacggaa agacc                                                        15

<210> 4
<211> 20
<212> DNA
<213> Artificial Sequence

<220>
<223> PCR probe

<400> 4
gtcctgatca atattaagct                                                   20

## Description

### BACKGROUND

### 1. Technical Field

The present invention relates to a method for discriminating the presence or absence of a mutation in a DNA.

### 2. Related Art

In recent years, a drug-resistant Mycoplasma pneumoniae which is resistant to macrolide antibiotics has been isolated from a patient.

In order to discriminate whether or not a mycoplasma is drug-resistant using a genetic testing method typified by a PCR (Polymerase Chain Reaction) method, it can be discriminated by examining mutations at positions 2063 and 2064 (normal type: A2063A and A2064A, highly drug-resistant type: A2063G, A2063C, A2064G, or A2064C, moderately drug-resistant type: A2063T) in the domain V region of an rRNA gene in the ribosome of the Mycoplasma pneumoniae (Rare Infectious Disease Diagnostic Techniques Workshop Report of Fiscal Year 2014, February 27, 2013 (Wednesday), Mycoplasma Infectious Diseases, Tsuyoshi Kenri, Department of Bacteriology II, National Institute of Infectious Diseases (NPL 1), Antimicrobial Agents and Chemotherapy pp. 1108-1109, Antibiotic Sensitivity of 40 Mycoplasma pneumoniae Isolates and Molecular Analysis of Macrolide-Resistant Isolates from Beijing, China, Fei Zhao, Min Lv, Xiaoxia Tao, Hui Huang, Binghua Zhang, Zhen Zhang, and Jianzhong Zhang (NPL 2)). It has been known that the normal type and the highly and moderately drug-resistant types are present at a ratio of 1: 9, and many of the Mycoplasma pneumoniae strains are drug-resistant.

On the other hand, an analysis of a mutant such as a drug-resistant strain has been performed so far by a sequence method, a PCR-RFLP method, a real-time PCR method, or the like.

However, in the case where the discrimination of a resistant strain is performed by a sequence method or the like in a related art, there is a problem that an experiment is complicated and also takes time.

### SUMMARY

An advantage of some aspects of the invention can be implemented as the following aspects.

An aspect of the invention is directed to a method for discriminating the presence or absence of a mutation having a plurality of mutated forms in a DNA including performing a thermal cycle for amplifying the DNA in the presence of one pair of primers which can bind to the DNA, a first probe which does not bind to the DNA having a mutation, can bind to the DNA having no mutation, and is labeled with a first fluorescent substance, and a second probe which can bind to both of the DNA having a mutation and the DNA having no mutation and is labeled with a second fluorescent substance which emits a light having a fluorescence wavelength different from that of a light emitted from the first fluorescent substance. A position of a base sequence in the strand of the DNA to which the first probe binds and a position of a base sequence in the strand of the DNA to which the second probe binds may be sandwiched between positions of base sequences in the strand of the DNA to which the pair of primers bind. The strand of the DNA to which the first probe binds may be a complementary strand of the DNA to which the second probe binds. The first probe may be a TaqMan probe, and the second probe may be a TaqMan probe. The amplification may be performed using a nucleic acid amplification reaction apparatus, and the nucleic acid amplification reaction apparatus may include: a fitting section capable of fitting a nucleic acid amplification reaction vessel filled with a nucleic acid amplification reaction mixture and a liquid which has a specific gravity smaller than that of the nucleic acid amplification reaction mixture and is immiscible with the nucleic acid amplification reaction mixture; a first heating section which heats a first region of the nucleic acid amplification reaction vessel; a second heating section which heats a second region of the nucleic acid amplification reaction vessel; and a driving mechanism which switches over between a first arrangement in which the first region is located lower than the second region in the direction of the gravitational force and a second arrangement in which the second region is located lower than the first region in the direction of the gravitational force. The DNA may be a DNA derived from Mycoplasma pneumoniae.

According to the aspect of the invention, a novel method for discriminating the presence or absence of a mutation in a DNA can be provided.

### BRIEF DESCRIPTION OF THE DRAWINGS

The invention will be described with reference to the accompanying drawings, wherein like numbers reference like elements.
FIG. 1 is a cross-sectional view of a nucleic acid amplification reaction vessel according to one embodiment of the invention. The arrow g indicates the direction of the gravitational force.
FIGS. 2A and 2B are perspective views of an up-and-down type PCR apparatus according to one embodiment of the invention. FIG. 2A shows a state in which a lid is closed and FIG. 2B shows a state in which the lid is opened.
FIG. 3 is an exploded perspective view of a main body of the up-and-down type PCR apparatus according to one embodiment of the invention.
FIGS. 4A and 4B are cross-sectional views schematically showing the cross section taken along the line A-A of FIG. 2A of the main body of the up-and-down type PCR apparatus according to one embodiment of the invention. FIG. 4A shows a first arrangement and FIG. 4B shows a second arrangement.
FIG. 5 is a view showing the positions of a normal type probe, a presence/absence probe, and a pair of primers according to one embodiment of the invention.
FIG. 6 shows graphs representing the results of discrimination between a normal strain and a resistant strain using the normal type probe and the presence/absence probe according to one embodiment of the invention.
FIG. 7 shows graphs representing the results of discrimination between a normal strain and a resistant strain using the normal type probe and the presence/absence probe according to one embodiment of the invention.
FIG. 8 shows graphs representing the results of discrimination between a normal strain and a resistant strain using the normal type probe and the presence/absence probe according to one embodiment of the invention.
FIG. 9 shows graphs representing the results of discrimination between a normal strain and a resistant strain using the normal type probe and the presence/absence probe according to one embodiment of the invention.
FIG. 10 shows graphs representing the results of discrimination between a normal strain and a resistant strain using the normal type probe and the presence/absence probe according to one embodiment of the invention.
FIG. 11 shows graphs representing the results of discrimination between a normal strain and a resistant strain using the normal type probe and the presence/absence probe according to one embodiment of the invention.
FIG. 12 shows a graph representing the results of discrimination between a normal strain and a resistant strain using the normal type probe and the presence/absence probe according to another embodiment of the invention.
FIG. 13 shows graphs representing the results of discrimination between a normal strain and a resistant strain using the normal type probe and the presence/absence probe according to still another embodiment of the invention.
FIG. 14 shows a graph representing the results of discrimination between a normal strain and a resistant strain using the normal type probe and the presence/absence probe according to yet still another embodiment of the invention.

### DESCRIPTION OF EXEMPLARY EMBODIMENTS

The object, features, and advantages of the invention as well as the idea thereof will be apparent to those skilled in the art from the description given herein, and the invention can be easily reproduced by those skilled in the art based on the description given herein. It is to be understood that the embodiments, specific examples, etc. of the invention described below are to be taken as preferred embodiments of the invention, and are presented for illustrative or explanatory purposes and are not intended to limit the invention. It is further apparent to those skilled in the art that various changes and modifications may be made based on the description given herein within the intent and scope of the invention disclosed herein.

### (1) Method for Discriminating Presence or Absence of Mutation in DNA

The discrimination method according to the invention is a method for discriminating the presence or absence of a mutation with a plurality of mutated forms in a DNA (for instance, a nucleotide at a certain position in the DNA can be mutated into more than one other nucleotide, as reflected in the below Table 1), wherein the presence or absence of a mutation is discriminated using a probe, e.g. a TaqMan probe, which binds to a DNA having no mutation and does not bind to a DNA having a mutation.

Hereinafter, one embodiment of the discrimination method according to the invention will be described by taking a DNA derived from a Mycoplasma pneumoniae strain as an example. A normal strain and a resistant strain are discriminated by using a probe, e.g. a TaqMan probe, which can bind to a DNA derived from a Mycoplasma pneumoniae strain having no drug resistant mutation (hereinafter referred to as "normal strain") and cannot bind to a DNA derived from a Mycoplasma pneumoniae strain having a drug resistant mutation (hereinafter referred to as "resistant strain"), and a TaqMan probe which can bind to both DNAs.

Mutations causing drug resistance in resistant strains are summarized in a table as follows.

**Table 1**

| | |
|---|---|
| Normal Strain | A2063A |
| | A2064A |
| Highly drug-resistant strain | A2063G |
| | A2063C |
| | A2064G |
| | A2064C |
| Moderately drug-resistant strain | A2063T |

In a related art, a normal strain and a resistant strain were discriminated by examining the base sequences of DNAs of both of the normal strain and the resistant strain. However, in the invention, a normal strain and a resistant strain can be discriminated more simply than in a related art by performing PCR using a probe, e.g. a TaqMan probe, which binds to a DNA of the normal strain and does not bind to a DNA of the resistant strain and a probe, e.g. a TaqMan probe, which binds to DNAs of both of the normal strain and the resistant strain.

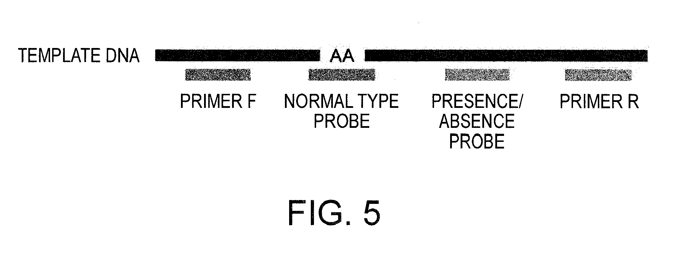

Specifically, as shown in FIG. 5, a probe, e.g. a TaqMan probe, which binds to a DNA when the bases at positions 2063 and 2064 are both A and is labeled with a first fluorescent substance (hereinafter referred to as "normal type probe") and a probe, e.g. a TaqMan probe, which binds to a DNA region having the same base sequence of the normal strain and the resistant strain and is labeled with a second fluorescent substance which emits a fluorescence wavelength different from that emitted from the first fluorescent substance (hereinafter referred to as "presence/absence probe") are used, and one pair of primers are located at positions so as to sandwich these probes, and then, e.g., TaqMan PCR is performed.

To the DNA of the normal strain, the normal type probe and the presence/absence probe can bind, and to the DNA of the resistant strain, the normal type probe cannot bind but the presence/absence probe can bind, and therefore, in the resistant strain, an increase in first fluorescence derived from the normal type probe is not observed.

In this embodiment, in the normal type probe, the length of the base sequences upstream and downstream of the bases at positions 2063 and 2064 can be arbitrarily designed as long as the normal type probe contains the bases at positions 2063 and 2064. The sequence of the presence/absence probe can be arbitrarily designed as long as the presence/absence probe binds to a sequence region having the same sequence of the normal type and the resistant type.

The DNA strands to which the normal type probe and the presence/absence probe bind may be the same DNA strand or may be DNA strands complementary to each other, but are preferably DNA strands complementary to each other.

The types of the fluorescent labels for the normal type probe and the presence/absence probe are not particularly limited, and known fluorescent labels can be used. The first fluorescent substance and the second fluorescent substance emit different fluorescence wavelengths, and a larger difference in the wavelength facilitates the discrimination, and therefore is preferred.

Also, the above-mentioned pair of primers can be arbitrarily designed as long as the primers are located at positions so as to sandwich the DNA regions to which the normal type probe and the presence/absence probe bind as shown in FIG. 5.

According to the method of the invention, even if a pathogen has a mutation with a plurality of mutated forms, it is not necessary to prepare probes according to the respective mutations like a related art, and the presence or absence of a resistant strain can be discriminated by only using the normal type probe and the presence/absence probe, and thus, the discrimination can be achieved more simply and at lower cost than in a related art.

### (2) Reaction Mixture to be used in Discrimination Method according to Invention

A reaction mixture to be used in the discrimination method according to the invention contains the above-mentioned normal type probe, presence/absence probe, and pair of primers, and may further contain other components necessary for a PCR reaction. Other components may include a buffer, a polymerase, dNTPs, MgCl₂, and the like. The DNA polymerase is not particularly limited, but is preferably a heat-resistant enzyme or an enzyme for use in PCR, and there are a great number of commercially available products, for example, Taq polymerase, Tfi polymerase, Tth polymerase, modified forms thereof, and the like, however, a DNA polymerase capable of performing hot start PCR is preferred. The concentration of dNTPs or a salt may be set to a concentration suitable for the enzyme to be used, however, the concentration of dNTPs may be set to generally 10 to 1000 µM, and preferably 100 to 500 %M, the concentration of Mg²⁺ may be set to generally 1 to 100 mM, and preferably 5 to 10 mM, and the concentration of Cl⁻ may be set to generally 1 to 2000 mM, and preferably 200 to 700 mM. The total ion concentration is not particularly limited, but may be higher than 50 mM, and is preferably higher than 100 mM, more preferably higher than 120 mM, further more preferably higher than 150 mM, and still further more preferably higher than 200 mM. The upper limit thereof is preferably 500 mM or less, more preferably 300 mM or less, and further more preferably 200 mM or less. Each oligonucleotide for the primer is used at 0.1 to 10 µM, and preferably at 0.1 to 1 µM. A reagent may contain a surfactant other than the above-mentioned components. The surfactant is not particularly limited, however, examples thereof include NP-40, Triton X-100, and Tween 20. The concentration of the surfactant is not particularly limited, but is preferably a concentration which does not inhibit the nucleic acid amplification reaction, and may be from 0.001% to 0.1% or less, and is preferably from 0.002% to 0.02%, and most preferably from 0.005% to 0.01%. A DNA to be discriminated and the above-mentioned reagent are mixed with each other to form a reaction mixture, and a nucleic acid amplification reaction can be performed using any known PCR apparatus.

### (3) Use of Method according to Invention and Reagent

The method according to the invention and the reagent can be used in an arbitrary nucleic acid amplification reaction apparatus, for example, a PCR apparatus.

The invention can be used for a DNA containing an arbitrary mutation with a plurality of mutated forms. For example, the invention can be used for discriminating the presence or absence of a pathogen having a mutation in the pathogen. The pathogen is not particularly limited as long as it causes a disease in living organisms, however, representative examples thereof include pathogenic microorganisms such as bacteria, viruses, rickettsiae, mycoplasma, Staphylococcus aureus, Streptococcus pneumoniae, influenza viruses, ESBL-producing bacteria, Neisseria gonorrhoeae, Mycobacterium tuberculosis, hemolytic streptococcus, Clostridium difficile, enteric bacteria, enterococci, Acinetobacter, Pseudomonas aeruginosa, Staphylococcus aureus, Campylobacter, Candida, nontyphoid Salmonella enterica, Salmonella typhi, Shigella dysenteriae, Group A Streptococcus, Group B Streptococcus, nontuberculous mycobacteria, spirochete, and fungi.

Further, microorganisms having an RNA such as low-susceptible influenza viruses and retroviruses can be test subjects. In such a case, by converting an RNA to a DNA by reverse transcription before a DNA is amplified, the method according to the invention can be applied.

### (4) Nucleic Acid Amplification Reaction Apparatus and Thermal Cycling Method

The PCR apparatus to which the method according to the invention is applied is not particularly limited. In one embodiment of the invention, a PCR apparatus (hereinafter referred to as "up-and-down PCR apparatus") described below is used.

### (i) Nucleic Acid Amplification Reaction Vessel

A nucleic acid amplification reaction vessel to be used in the method according to the invention is a nucleic acid amplification reaction vessel which has a hermetically sealed vessel containing a reaction mixture and a liquid having a specific gravity different from that of the reaction mixture and phase-separated from the reaction mixture, wherein the reaction mixture is in the form of a liquid droplet and the liquid contains an oil and an additive.

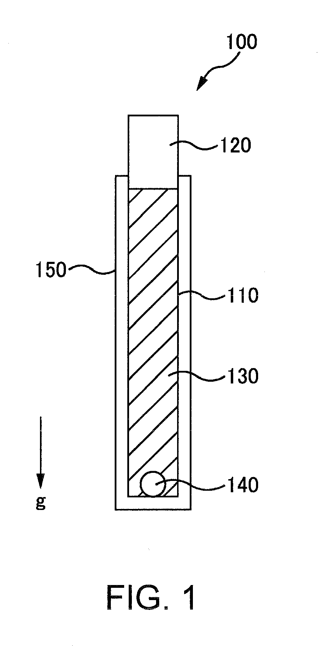

FIG. 1 is a cross-sectional view of a nucleic acid amplification reaction vessel 100. FIG. 1 shows a state in which a reaction mixture is placed in the nucleic acid amplification reaction vessel.

The nucleic acid amplification reaction vessel 100 to be used in the invention is configured to include a vessel 150 and a sealing section 120. The size and shape of the nucleic acid amplification reaction vessel 100 are not particularly limited, but may be designed in consideration of, for example, at least one of the amount of a liquid 130 which is immiscible with a reaction mixture 140 as exemplified by the above-mentioned reaction mixture, the thermal conductivity thereof, the shapes of the vessel 150 and the sealing section 120, and the ease of handling thereof.

The vessel 150 of the nucleic acid amplification reaction vessel 100 can be formed from a transparent material. According to this, the movement of the reaction mixture 140 in the vessel 150 can be observed from the outside of the nucleic acid amplification reaction vessel 100, or the vessel 150 can be used in an application in which the measurement or the like is performed from the outside of the vessel 150 such as real-time PCR. The term "transparent" as used herein refers to a condition in which the visibility can be ensured to such an extent that the reaction mixture 140 in the vessel 150 can be observed from the outside of the vessel 150, and it is not necessary that the entire nucleic acid amplification reaction vessel 100 should be transparent as long as this condition is met.

The application of the nucleic acid amplification reaction vessel 100 is not particularly limited, however, for example, in the case where the nucleic acid amplification reaction vessel 100 is used in an application with a fluorescence measurement such as real-time PCR, the vessel 150 is desirably formed from a material with a low autofluorescence. The vessel 150 is preferably formed from a material which can withstand heating in PCR. Further, the material of the vessel 150 is preferably a material, on which nucleic acids or proteins are less adsorbed, and which does not inhibit the enzymatic reaction by a polymerase or the like. The material which satisfies these conditions is not particularly limited, and for example, polypropylene, polyethylene, a cycloolefin polymer (for example, ZEONEX (registered trademark) 480R), a heat-resistant glass (for example, PYREX (registered trademark) glass), or the like, or a composite material thereof may be used, however, polypropylene is preferred.

In the nucleic acid amplification reaction vessel 100 shown in FIG. 1, the vessel 150 is formed into a cylindrical shape, and the direction of the center axis (the vertical direction in Fig. 1) coincides with the longitudinal direction. The vessel 150 used here is preferably a tube and may be a tube for a microcentrifuge or a tube designed for PCR. Since the vessel 150 has a shape with a longitudinal direction, in other words, an elongated shape, for example, in the case where the temperature of the nucleic acid amplification reaction vessel 100 is controlled so that regions having different temperatures are formed in the liquid 130 in the vessel 150 using an up-and-down type thermal cycler, which will be described later, the distance between the regions having different temperatures is easily increased. According to this, it becomes easy to control the temperature of the liquid 130 to be different from region to region in the vessel 150, and therefore, a thermal cycle suitable for PCR can be realized. The "up-and-down type thermal cycler" is an apparatus which realizes a thermal cycle by forming at least two temperature regions in a liquid filled in the vessel 150 and allowing the reaction mixture 140 which is phase-separated from the liquid to move reciprocatingly between these temperature regions.

The shape of the vessel 150 is not particularly limited as long as it has a longitudinal direction, however, in the case where the vessel 150 is used for the up-and-down type PCR apparatus, it is preferred that the shape is a substantially cylindrical shape and the ratio of the inner diameter D to the length L in the longitudinal direction is in the range of 1:5 to 1.5:20. It is more preferred that the inner diameter D is from 1.5 to 2 mm, and the length L is from 10 to 20 mm.

The vessel 150 has an opening section and the sealing section 120 which seals the opening section, and in the vessel 150, the reaction mixture 140 and the liquid 130 which has a specific gravity different from that of the reaction mixture 140 and is phase-separated from the reaction mixture 140 are contained. It is preferred that in the case where the opening section is sealed by the sealing section 120, air does not remain in the vessel 150. It is because if an air bubble remains in the vessel 150, the movement of the reaction mixture 140 may be hindered. The sealing section 120 can be formed from the same material as that of the vessel 150. The structure of the sealing section 120 may be any as long as it can hermetically seal the vessel 150, and can be a structure of, for example, a screw cap, a plug, an inlay, or the like. In FIG. 1, the sealing section 120 has a structure of a screw cap.

The reaction mixture 140 may further contain a surfactant. The surfactant is not particularly limited, however, examples thereof include NP-40, Triton X-100, and Tween 20. The concentration of the surfactant is not particularly limited, but is preferably a concentration which does not inhibit the nucleic acid amplification reaction, and may be from 0.001% to 0.1% or less, and is preferably from 0.002% to 0.02%, and most preferably from 0.005% to 0.01%. The surfactant may be a carry-over from a stock solution of the enzyme described above, however, a surfactant solution may be added to the reaction mixture 140 independently of the stock solution of the enzyme.

By using a liquid which is immiscible with the reaction mixture 140 as the liquid 130, when the reaction mixture 140 is placed in the vessel 150, the reaction mixture 140 and the liquid 130 are phase-separated from each other, and therefore, the reaction mixture 140 can be formed into a liquid droplet in the liquid 130. In this manner, the reaction mixture 140 is maintained in the form of a liquid droplet in the liquid 130.

The liquid 130 is preferably a liquid having a specific gravity smaller than that of the reaction mixture 140. In this case, when the reaction mixture 140 is placed in the liquid 130, the liquid droplet of the reaction mixture 140 has a specific gravity larger than that of the liquid 130, and therefore moves in the direction of the gravitational force by the action of gravity. Further, the liquid 130 may be a liquid having a specific gravity larger than that of the reaction mixture 140. In this case, the liquid droplet of the reaction mixture 140 has a specific gravity smaller than that of the liquid 130, and therefore moves in the direction opposite to the direction of the gravitational force by the action of gravity.

The liquid 130 preferably contains an oil, and for example, a silicone oil or a mineral oil can be used. Here, the "silicone" means an oiligomer or a polymer having a siloxane bond as a main skeleton. In this specification, among silicones, a silicone in the form of a liquid in a temperature range in which the silicone is used in a thermal cycling treatment is particularly referred to as "silicone oil". Further, in this specification, an oil which is purified from petroleum and is in the form of a liquid in a temperature range in which the oil is used in a thermal cycling treatment is referred to as "mineral oil". These oils have high stability against heat, and for example, products having a viscosity of 5 x 10³ Nsm⁻² or less are also easily available, and therefore, these oils are preferred for use in up-and-down type PCR. The viscosity of the oil is not particularly limited, but is preferably 90 cs or less, more preferably 70 cs or less, further more preferably 50 cs or less, and still further more preferably 30 cs or less. In this manner, the viscosity of the oil is preferably smaller, and according to this, when the reaction mixture goes down as described later, the reaction mixture can go down more smoothly.

Examples of the silicone oil include dimethyl silicone oils such as KF-96L-0.65cs, KF-96L-lcs, KF-96L-2cs, KF-96L-5cs (manufactured by Shin-Etsu Silicone Co., Ltd.), SH200 C FLUID 5 CS (manufactured by Dow Corning Toray Co. , Ltd.), TSF451-5A, and TSF451-10 (manufactured by Momentive Performance Materials Japan LLC). Examples of the mineral oil include oils containing alkane having about 14 to 20 carbon atoms as a principal component, and specific examples thereof include n-tetradecane, n-pentadecane, n-hexadecane, n-heptadecane, n-octadecane, n-nonadecane, and n-tetracosane.

The liquid 130 may contain an additive. As the additive, for example, a modified silicone oil such as X-22-160AS, X-22-3701E, KF-857, KF-859, KF-862, KF-867, KF-6017, or KF-8005 (Shin-Etsu Silicone Co. , Ltd.), a silicone resin such as SR1000, SS4230, SS4267, or YR3370 (Momentive Performance Materials, Inc.), a fluoro-modified silicone resin such as XS66-C1191 (Momentive Performance Materials, Inc.), or the like, and other than these, a modified silicone oil such as TSF4703, TSF4708, XF42-C5196, or XF42-C5197 (Momentive Performance Materials, Inc.) can be used. The concentration of the additive is not particularly limited, but can be determined in consideration of the structure, material, shape, or the like of the vessel. For example, the concentration thereof is preferably 1% (v/v) or more and 50% (v/v) or less, more preferably 2% (v/v) or more and 20% (v/v) or less, and further more preferably 5% (v/v).

### (ii) Up-and-down type Nucleic Acid Amplification Reaction Apparatus

In this embodiment, as the nucleic acid amplification reaction vessel to be used for performing a nucleic acid amplification reaction, a nucleic acid amplification reaction vessel in the form of a tube (nucleic acid amplification reaction tube) 100 is used. Hereinafter, by taking PCR as one example of the nucleic acid amplification reaction, one example of an up-and-down type nucleic acid amplification reaction apparatus (hereinafter also referred to as "up-and-down type PCR apparatus") suitable for the nucleic acid amplification reaction vessel 100 will be described in detail.

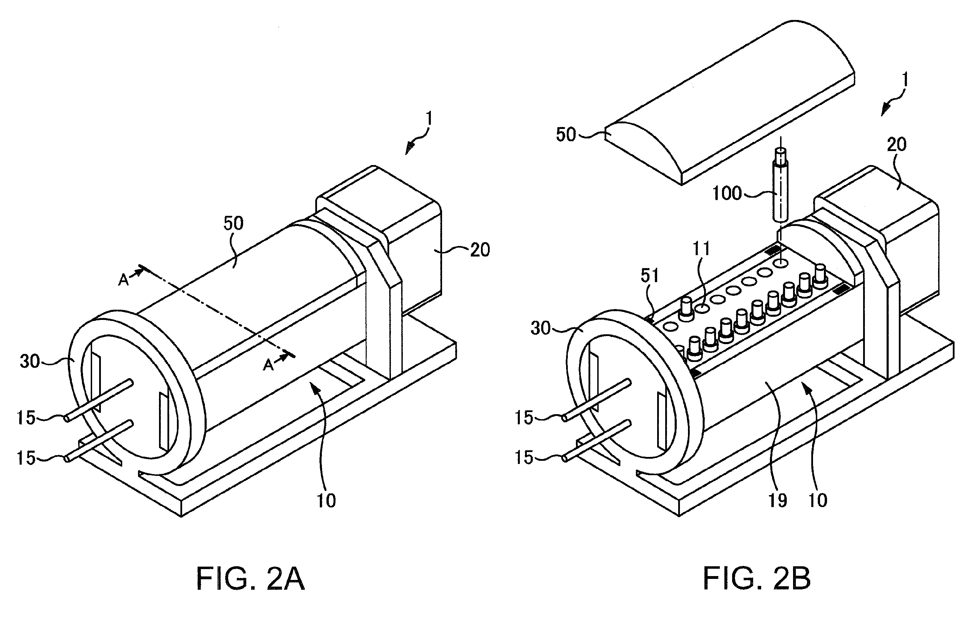

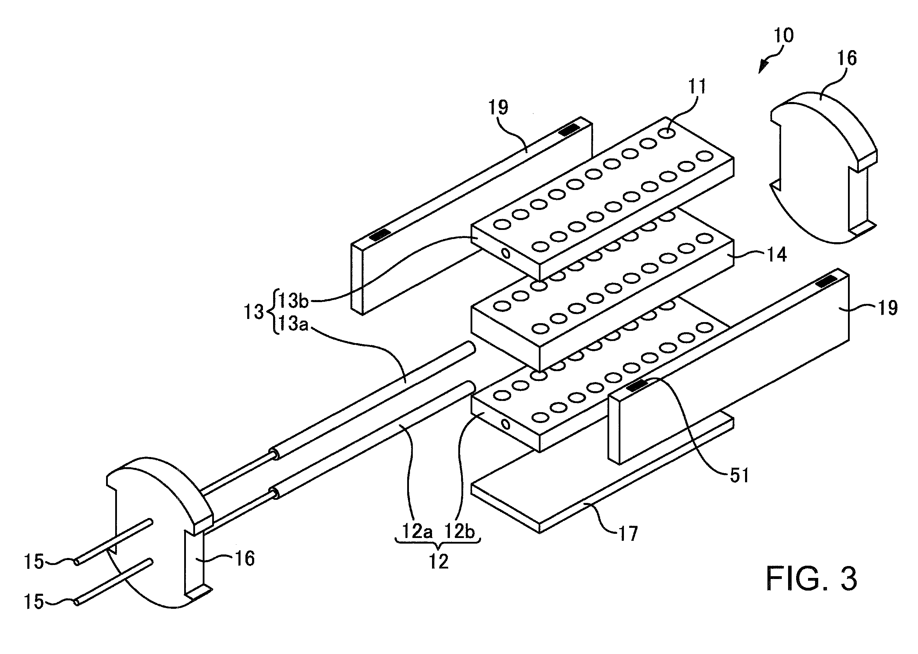

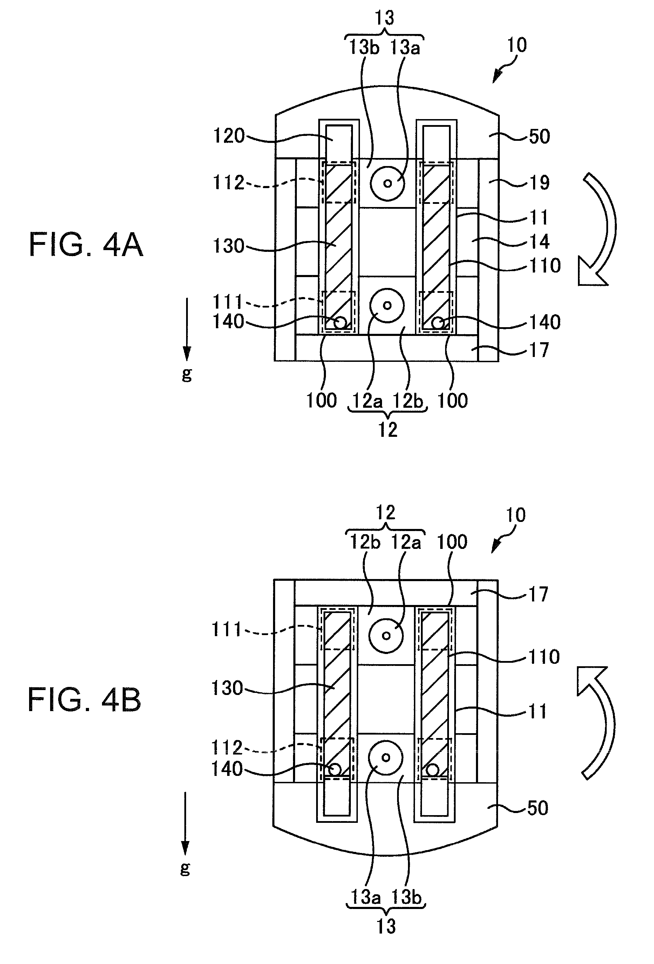

FIGS. 2A and 2B show one example of an up-and-down type PCR apparatus 1. FIG. 2A shows a state in which a lid 50 of the up-and-down type PCR apparatus 1 is closed, and FIG. 2B shows a state in which the lid 50 of the up-and-down type PCR apparatus 1 is opened and the nucleic acid amplification reaction vessel 100 is fitted in a fitting section 11. FIG. 3 is an exploded perspective view of a main body 10 of the up-and-down type PCR apparatus 1 according to the embodiment. FIGS. 4A and 4B are cross-sectional views schematically showing the cross section taken along the line A-A of FIG. 2A of the main body 10 of the up-and-down type PCR apparatus 1 according to the embodiment.

This up-and-down type PCR apparatus 1 includes the main body 10 and a driving mechanism 20 as shown in FIG. 2A. As shown in FIG. 3, the main body 10 includes the fitting section 11, a first heating section 12, and a second heating section 13. A spacer 14 is provided between the first heating section 12 and the second heating section 13. In the main body 10 of this embodiment, the first heating section 12 is disposed on the bottom plate 17 side, and the second heating section 13 is disposed on the lid 50 side. In the main body 10 of this embodiment, the first heating section 12, the second heating section 13, and the spacer 14 are fixed by a flange 16, the bottom plate 17, and a fixing plate 19.

The fitting section 11 is configured such that the nucleic acid amplification reaction vessel 100, which will be described later, is fitted therein. As shown in FIG. 2B and FIG. 3, the fitting section 11 of this embodiment has a slot structure in which the nucleic acid amplification reaction vessel 100 is inserted and fitted, and is configured such that the nucleic acid amplification reaction vessel 100 is inserted into a hole penetrating a first heat block 12b of the first heating section 12, the spacer 14, and a second heat block 13b of the second heating section 13. The number of the fitting sections 11 may be more than one, and in the example shown in FIG. 2B, twenty fitting sections 11 are provided for the main body 10.

This up-and-down type PCR apparatus 1 includes a structure in which the nucleic acid amplification reaction vessel 100 is held at a predetermined position with respect to the first heating section 12 and the second heating section 13. More specifically, as shown in FIGS. 4A and 4B, in a flow channel 110 constituting the nucleic acid amplification reaction vessel 100, which will be described later, a first region 111 can be heated by the first heating section 12 and a second region 112 can be heated by the second heating section 13. In this embodiment, a structure that defines the position of the nucleic acid amplification reaction vessel 100 is the bottom plate 17, and as shown in FIG. 4A, by inserting the nucleic acid amplification reaction vessel 100 to a position where the vessel is in contact with the bottom plate 17, the nucleic acid amplification reaction vessel 100 can be held at a predetermined position with respect to the first heating section 12 and the second heating section 13.

When the nucleic acid amplification reaction vessel 100 is fitted in the fitting section 11, the first heating section 12 heats the first region 111 of the nucleic acid amplification reaction vessel 100, which will be described later, to a first temperature. In the example shown in FIG. 4A, in the main body 10, the first heating section 12 is disposed at a position where the first region 111 of the nucleic acid amplification reaction vessel 100 is heated.

The first heating section 12 may include a mechanism that generates heat and a member that transfers the generated heat to the nucleic acid amplification reaction vessel 100. In the example shown in FIG. 3, the first heating section 12 includes a first heater 12a and a first heat block 12b. In this embodiment, the first heater 12a is a cartridge heater and is connected to an external power source (not shown) through a conductive wire 15. The first heater 12a is inserted into the first heat block 12b, and the first heat block 12b is heated by heat generated by the first heater 12a. The first heat block 12b is a member that transfers heat generated by the first heater 12a to the nucleic acid amplification reaction vessel 100. In this embodiment, the first heat block 12b is a block made of aluminum.

When the nucleic acid amplification reaction vessel 100 is fitted in the fitting section 11, the second heating section 13 heats the second region 112 of the nucleic acid amplification reaction vessel 100 to a second temperature different from the first temperature. In the example shown in FIG. 4A, in the main body 10, the second heating section 13 is disposed at a position where the second region 112 of the nucleic acid amplification reaction vessel 100 is heated. As shown in FIG. 2, the second heating section 13 includes a second heater 13a and the second heat block 13b. The second heating section 13 is configured in the same manner as the first heating section 12 except that the region of the nucleic acid amplification reaction vessel 100 to be heated and the heating temperature are different from those for the first heating section 12.

In this embodiment, the temperatures of the first heating section 12 and the second heating section 13 are controlled by a temperature sensor (not shown) and a control section (not shown), which will be described later. The temperatures of the first heating section 12 and the second heating section 13 are preferably set so that the nucleic acid amplification reaction vessel 100 is heated to a desired temperature. In this embodiment, by controlling the first heating section 12 at the first temperature and the second heating section 13 at the second temperature, the first region 111 of the nucleic acid amplification reaction vessel 100 can be heated to the first temperature, and the second region 112 can be heated to the second temperature. The temperature sensor in this embodiment is a thermocouple.

The driving mechanism 20 is a mechanism that controls the fitting section 11, the first heating section 12, and the second heating section 13, and by the driving mechanism, the arrangement of the first region and the second region is controlled. In this embodiment, the driving mechanism 20 includes a motor (not shown) and a drive shaft (not shown), and the drive shaft is connected to the flange 16 of the main body 10. The drive shaft in this embodiment is provided perpendicular to the longitudinal direction of the fitting section 11, and when the motor is activated, the main body 10 is rotated about the drive shaft as the axis of rotation.

The up-and-down type PCR apparatus 1 of this embodiment includes the control section (not shown). The control section controls at least one of the first temperature, the second temperature, a first period, a second period, and the cycle number of thermal cycles, which will be described later. In the case where the control section controls the first period or the second period, the control section controls the operation of the driving mechanism 20, thereby controlling the period in which the fitting section 11, the first heating section 12, and the second heating section 13 are held in a predetermined arrangement. The control section may have mechanisms different from item to item to be controlled, or may control all items collectively. However, the control section in the up-and-down type PCR apparatus 1 of this embodiment is an electronic control system and controls all of the above-mentioned items. The control section of this embodiment includes a processor such as a CPU (not shown) and a storage device such as an ROM (Read Only Memory) or an RAM (Random Access Memory). In the storage device, various programs, data, etc. for controlling the above-mentioned respective items are stored. Further, the storage device has a work area for temporarily storing data in processing, processing results, etc. of various processes.

As shown in the example of FIG. 3 and FIG. 4A, in the main body 10 of this embodiment, the spacer 14 is provided between the first heating section 12 and the second heating section 13. The spacer 14 of this embodiment is a member that holds the first heating section 12 or the second heating section 13. In this embodiment, the spacer 14 is a heat insulating material, and in the example shown in FIG. 4A, the fitting section 11 penetrates the spacer 14.

The main body 10 of this embodiment includes the fixing plate 19. The fixing plate 19 is a member that holds the fitting section 11, the first heating section 12, and the second heating section 13. In the example shown in FIG. 2B and FIG. 3, two fixing plates 19 are fitted in the flanges 16, and the first heating section 12, the second heating section 13, and the bottom plate 17 are fixed by the fixing plates 19.

The up-and-down type PCR apparatus 1 of this embodiment includes the lid 50. In the example shown in FIG. 2A and FIG. 4A, the fitting section 11 is covered with the lid 50. The lid 50 may be fixed to the main body 10 by a fixing section 51. In this embodiment, the fixing section 51 is a magnet. As shown in the example of FIG. 2B and FIG. 3, a magnet is provided on a surface of the main body 10 which comes into contact with the lid 50. Although not shown in FIG. 2B and FIG. 3, a magnet is provided also for the lid 50 at a place where the magnet of the main body 10 comes into contact. When the fitting section 11 is covered with the lid 50, the lid 50 is fixed to the main body 10 by a magnetic force.

It is preferred that the fixing plate 19, the bottom plate 17, the lid 50, and the flange 16 are formed using a heat insulating material.

### (iii) Thermal Cycling Treatment Using Up-and-down type PCR Apparatus

FIGS. 4A and 4B are cross-sectional views schematically showing the cross section taken along the line A-A of FIG. 2A of the up-and-down type PCR apparatus 1. FIGS. 4A and 4B show a state in which the nucleic acid amplification reaction vessel 100 is fitted in the up-and-down type PCR apparatus 1. FIG. 4A shows a first arrangement and FIG. 4B shows a second arrangement. Hereinafter, a thermal cycling treatment using the up-and-down type PCR apparatus 1 according to the embodiment in the case of using the nucleic acid amplification reaction vessel 100 will be described.

As shown in the example of FIG. 1, the nucleic acid amplification reaction vessel 100 according to the embodiment includes a flow channel 110 and a sealing section 120. The flow channel 110 is filled with a reaction mixture 140 and an oil serving as a liquid 130, which has a specific gravity smaller than that of the reaction mixture 140 and is immiscible with the reaction mixture 140, and sealed with the sealing section 120.

The flow channel 110 is formed such that the reaction mixture 140 moves in close proximity to opposed inner walls. Here, the phrase "opposed inner walls" of the flow channel 110 refers to two regions of a wall surface of the flow channel 110 having an opposed positional relationship. The phrase "in close proximity to" refers to a state in which the distance between the reaction mixture 140 and the wall surface of the flow channel 110 is close, and includes a case where the reaction mixture 140 is in contact with the wall surface of the flow channel 110. Therefore, the phrase "the reaction mixture 140 moves in close proximity to opposed inner walls" refers to that "the reaction mixture 140 moves in a state of being close in distance to both of the two regions of a wall surface of the flow channel 110 having an opposed positional relationship", that is, the reaction mixture 140 moves along the opposed inner walls.

In the example shown in Fig. 1, the outer shape of the nucleic acid amplification reaction vessel 100 is a cylindrical shape, and the flow channel 110 is formed in the direction of the center axis (the vertical direction in Fig. 1) therein. The shape of the flow channel 110 is a cylindrical shape having a circular cross section perpendicular to the longitudinal direction of the flow channel 110, that is, perpendicular to the direction in which the reaction mixture 140 moves in a region in the flow channel 110 (this cross section is defined as the "cross section" of the flow channel 110). Therefore, in the nucleic acid amplification reaction vessel 100 of this embodiment, the opposed inner walls of the flow channel 110 are regions including two points on the wall surface of the flow channel 110 constituting the diameter of the cross section of the flow channel 110, and the reaction mixture 140 moves in the longitudinal direction of the flow channel 110 along the opposed inner walls.

The first region 111 of the nucleic acid amplification reaction vessel 100 is a partial region of the flow channel 110 which is heated to the first temperature by the first heating section 12. The second region 112 is a partial region of the flow channel 110, which is different from the first region 111, and is heated to the second temperature by the second heating section 13. In the nucleic acid amplification reaction vessel 100 of this embodiment, the first region 111 is a region including one end portion in the longitudinal direction of the flow channel 110, and the second region 112 is a region including the other end portion in the longitudinal direction of the flow channel 110. In the example shown in FIGS. 4A and 4B, a region surrounded by the dotted line including an end portion on the proximal side of the sealing section 120 of the flow channel 110 is the second region 112, and a region surrounded by the dotted line including an end portion on the distal side of the sealing section 120 is the first region 111.

As shown in FIG. 1, the flow channel 110 contains the reaction mixture 140 and the oil serving as the liquid 130 immiscible with the reaction mixture 140. The liquid 130 and the reaction mixture 140 are prepared according to the description of the above (i) Nucleic Acid Amplification Reaction Vessel.

Hereinafter, with reference to FIGS. 4A and 4B, the thermal cycling treatment using the up-and-down type PCR apparatus 1 according to the embodiment will be described. In

FIGS. 4A and 4B, the direction indicated by the arrow g (in the downward direction in the drawing) is the direction of the gravitational force. In this embodiment, a case where shuttle PCR (two-stage temperature PCR) is performed as an example of the thermal cycling treatment will be described. The respective steps described below show one example of the thermal cycling treatment, and according to need, the order of the steps may be changed, two or more steps may be performed continuously or concurrently, or a step may be added.

The shuttle PCR is a method of amplifying a nucleic acid in a reaction mixture by subjecting the reaction mixture to a two-stage temperature treatment at a high temperature and a low temperature repeatedly. In the treatment at a high temperature, denaturation of a double-stranded DNA occurs and in the treatment at a low temperature, annealing (a reaction in which a primer binds to a single-stranded DNA) and elongation (a reaction in which a complementary strand to the DNA is synthesized by using the primer as a starting point) occur.

In general, in shuttle PCR, the high temperature is a temperature between 80°C and 100°C and the low temperature is a temperature between 50°C and 70°C. The treatments at the respective temperatures are performed for a predetermined period, and a period in which the reaction mixture is maintained at a high temperature is generally shorter than a period in which the reaction mixture is maintained at a low temperature. For example, the period of the treatment at a high temperature may be set to about 1 to 10 seconds, and the period of the treatment at a low temperature may be set to about 10 to 60 seconds, or a period longer than this range may be adopted depending on the condition of the reaction.

The appropriate period, temperature, cycle number (the number of repetitions of the treatment at a high temperature and the treatment at a low temperature) vary depending on the type or amount of a reagent to be used, and therefore, it is preferred to determine an appropriate protocol in consideration of the type of a reagent or the amount of the reaction mixture 140 before performing the reaction.

First, the nucleic acid amplification reaction vessel 100 is fitted in the fitting section 11. In this embodiment, after the reaction mixture 140 is introduced into the flow channel 110 previously filled with the liquid 130, the nucleic acid amplification reaction vessel 100 is sealed with the sealing section 120, and then fitted in the fitting section 11. The introduction of the reaction mixture 140 can be performed using a micropipette, an ink-jet dispenser, or the like. In a state in which the nucleic acid amplification reaction vessel 100 is fitted in the fitting section 11, the first heating section 12 is in contact with the nucleic acid amplification reaction vessel 100 at a position including the first region 111 and the second heating section 13 is in contact with the nucleic acid amplification reaction vessel 100 at a position including the second region 112.

Here, the arrangement of the fitting section 11, the first heating section 12, and the second heating section 13 is the first arrangement. As shown in FIG. 4A, in the first arrangement, the first region 111 of the nucleic acid amplification reaction vessel 100 is located in a lowermost portion of the flow channel 110 in the direction of the gravitational force. In the first arrangement, the first region 111 is located in a lowermost portion of the flow channel 110 in the direction of the gravitational force, and therefore, the reaction mixture 140 having a specific gravity larger than that of the liquid 130 is located in the first region 111. In this embodiment, after the nucleic acid amplification reaction vessel 100 is fitted in the fitting section 11, the fitting section 11 is covered with the lid 50, and then the up-and-down type PCR apparatus 1 is operated.

Subsequently, the nucleic acid amplification reaction vessel 100 is heated by the first heating section 12 and the second heating section 13. The first heating section 12 and the second heating section 13 heat different regions of the nucleic acid amplification reaction vessel 100 to different temperatures. That is, the first heating section 12 heats the first region 111 to the first temperature, and the second heating section 13 heats the second region 112 to the second temperature. According to this, a temperature gradient in which the temperature gradually changes between the first temperature and the second temperature is formed between the first region 111 and the second region 112 of the flow channel 110. Here, a temperature gradient in which the temperature decreases from the first region 111 to the second region 112 is formed. The thermal cycling treatment of this embodiment is shuttle PCR, and therefore, the first temperature is set to a temperature suitable for the denaturation of a double-stranded DNA, and the second temperature is set to a temperature suitable for annealing and elongation.

Since the arrangement of the fitting section 11, the first heating section 12, and the second heating section 13 is the first arrangement, when the nucleic acid amplification reaction vessel 100 is heated, the reaction mixture 140 is heated to the first temperature. When the first period has elapsed, the main body 10 is driven by the driving mechanism 20, and the arrangement of the fitting section 11, the first heating section 12, and the second heating section 13 is switched over from the first arrangement to the second arrangement. The second arrangement is an arrangement in which the second region 112 is located in a lowermost portion of the flow channel 110 in the direction of the gravitational force. In other words, the second region 112 is a region located in a lowermost portion of the flow channel 110 in the direction of the gravitational force when the fitting section 11, the first heating section 12, and the second heating section 13 are placed in a predetermined arrangement different from the first arrangement. In the up-and-down type PCR apparatus 1 of this embodiment, under the control of the control section, the driving mechanism 20 rotatively drives the main body 10. When the flanges 16 are rotatively driven by the motor by using the drive shaft as the axis of rotation, the fitting section 11, the first heating section 12, and the second heating section 13 which are fixed to the flanges 16 are rotated. Since the drive shaft is a shaft extending in the direction perpendicular to the longitudinal direction of the fitting section 11, when the drive shaft is rotated by the activation of the motor, the fitting section 11, the first heating section 12, and the second heating section 13 are rotated. In the example shown in FIGS. 4A and 4B, the main body 10 is rotated at 180°. By doing this, the arrangement of the fitting section 11, the first heating section 12, and the second heating section 13 is switched over from the first arrangement to the second arrangement.

Here, the positional relationship between the first region 111 and the second region 112 in the direction of the gravitational force is opposite to that of the first arrangement, and therefore, the reaction mixture 140 moves from the first region 111 to the second region 112 by the action of gravity. When the operation of the driving mechanism 20 is stopped after the arrangement of the fitting section 11, the first heating section 12, and the second heating section 13 has reached the second arrangement, the fitting section 11, the first heating section 12, and the second heating section 13 are held in the second arrangement. When the second period has elapsed in the second arrangement, the main body is rotated again. A nucleic acid amplification reaction is performed by rotation while switching over between the first arrangement and the second arrangement in this manner until completion of a predetermined number of cycles. An operation in which the first arrangement and the second arrangement are switched over once is defined as one cycle.

A period in which the nucleic acid amplification reaction mixture 140 moves from the first region to the second region can be arbitrarily set, but is preferably shorter. For example, the period may be 5 seconds or less, but is more preferably 2 seconds or less, and most preferably 1 second or less.

By performing this thermal cycling treatment using the above-mentioned nucleic acid amplification reaction vessel and up-and-down type PCR apparatus, the temperature of the reaction mixture in the form of a small liquid droplet can be quickly changed. For example, in shuttle PCR, a period in which the temperature of the reaction mixture is decreased from the high temperature to the set temperature suitable for annealing and elongation or a period in which the temperature of the reaction mixture is increased from the set temperature suitable for annealing and elongation to the high temperature is very short, and therefore, a period in which the temperature of the reaction mixture is outside the temperature range in which annealing and elongation can be performed is also very short.

### Examples

Hereinafter, the invention will be described in more detail by showing Examples, however, the invention is not limited to Examples. In the following Examples, the up-and-down type PCR apparatus and the thermal cycling method described in the embodiment were used.

### Example 1

### (1) Preparation of Reaction Mixture

25 copies of each of the plasmids derived from a normal Mycoplasma pneumoniae strain (hereinafter referred to as "normal strain") and a drug-resistant Mycoplasma pneumoniae strain (hereinafter referred to as "resistant strain") were prepared. The following 6 types of plasmids were used.
(i) Normal type: The bases at positions 2063 and 2064 of 23S rRNA in the ribosome of a Mycoplasma pneumoniae strain are "A" and "A", respectively.
(ii) Resistant type 1: The bases at positions 2063 and 2064 of 23S rRNA in the ribosome of a Mycoplasma pneumoniae strain are "G" and "A", respectively.
(iii) Resistant type 2: The bases at positions 2063 and 2064 of 23S rRNA in the ribosome of a Mycoplasma pneumoniae strain are "C" and "A", respectively.
(iv) Resistant type 3: The bases at positions 2063 and 2064 of 23S rRNA in the ribosome of a Mycoplasma pneumoniae strain are "A" and "G", respectively.
(v) Resistant type 4: The bases at positions 2063 and 2064 of 23S rRNA in the ribosome of a Mycoplasma pneumoniae strain are "A" and "C", respectively.
(vi) Resistant type 5: The bases at positions 2063 and 2064 of 23S rRNA in the ribosome of a Mycoplasma pneumoniae strain are "A" and "T", respectively.

A reaction mixture was prepared by mixing any of the above-mentioned plasmids and the following reagent for discrimination with each other. The composition of the reaction mixture is as follows. The liquid amount of each component is a liquid amount per 10 µL of the total amount of the reaction mixture.

| | |
|---|---|
| Platinum Taq (polymerase) | 0.4 µL |
| dNTPs (10 mM) | 0.5 µL |
| 5x buffer (*) | 2.0 µL |
| Forward primer (20 µM) | 0.8 µL |
| Reverse primer (20 µM) | 0.8 µL |
| Normal type probe (TaqMan probe, 10 µM) | 0.6 µL |
| Presence/absence probe (TaqMan probe, 10 µM) | 0.6 µL |
| Template DNA | 1.0 µL |
| Water | up to 10.0 µL |

| | |
|---|---|
| * Composition of 5x buffer: MgCl₂: 25 mM, Tris-HCl (pH 9.0): 250 mM, KCl: 125 mM | |

The sequences of the primers and the probes are as follows. The normal type probe and the presence/absence probe were designed such that the DNA strands to which the normal type probe and the presence/absence probe bind are DNA strands complementary to each other. As the fluorescent dyes for labeling the normal type probe and the presence/absence probe, FAM and Cy5.5 were used, respectively.
Forward primer: 5' ATCCAGGTACGGGTGAAGACAC 3' (SEQ ID NO: 1)
Reverse primer: 5' CGCATCAACAAGTCCTAGCGAAC 3' (SEQ ID NO: 2)
Normal type probe: 5' FAM-CGGGACGGA(2063)A(2064)AGACC-BHQ1 3' (SEQ ID NO: 3)
Presence/absence probe: 5' Cyanine5.5-GTCCTGATCAATATTAAGCT-BHQ3 3' (SEQ ID NO: 4)

### (2) Conditions for PCR

By using the up-and-down PCR apparatus, a nucleic acid amplification reaction was performed under the following conditions using 1.4 µL of the reaction mixture.
Hot start: 98°C, 10 sec
(1 cycle)
Thermal denaturation: 98°C, 4 sec
Annealing/elongation: 54°C, 6 sec
(50 cycles)

The period required for the nucleic acid amplification reaction was 8 minutes.

### (3) Discrimination of Normal Strain and Resistant Strain

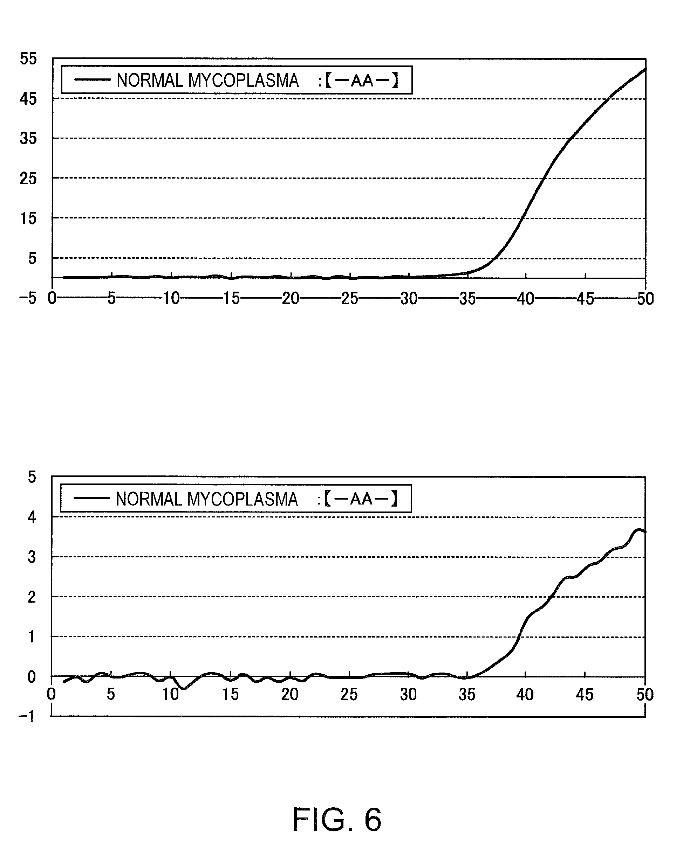

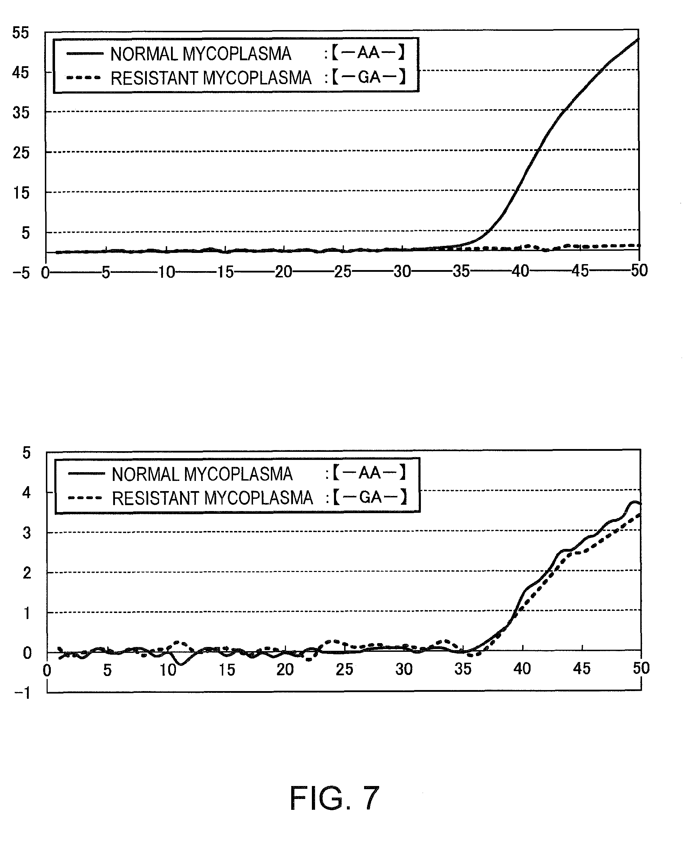

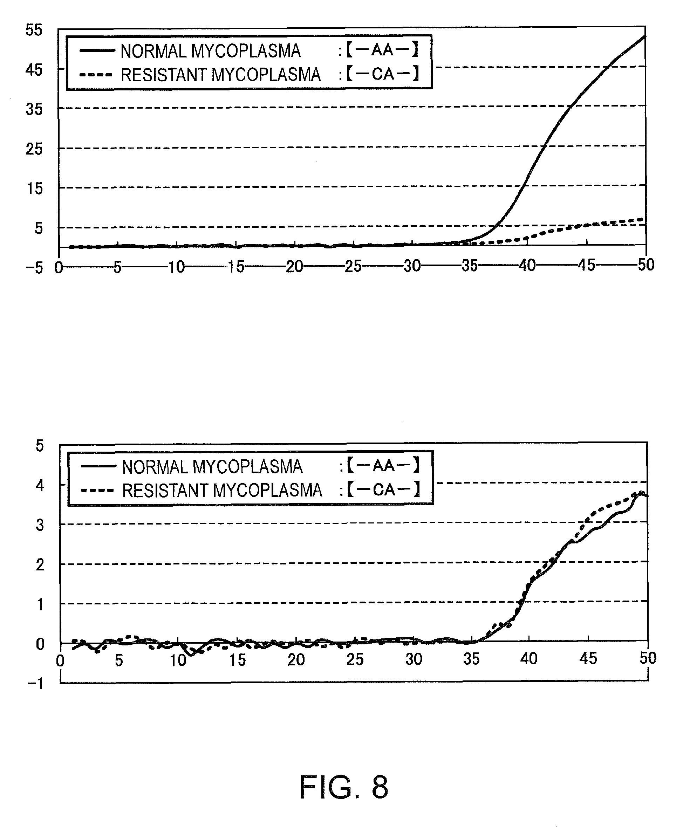

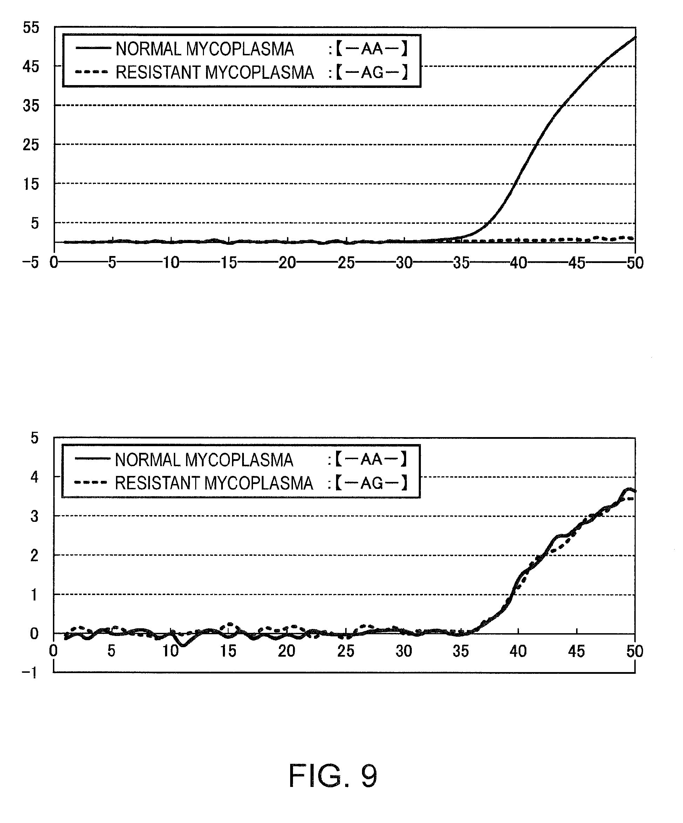

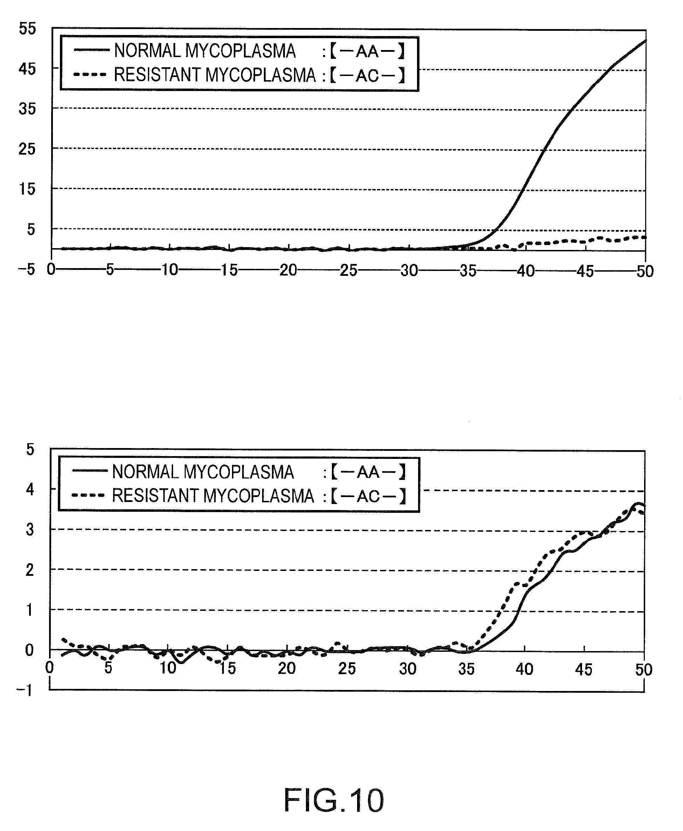

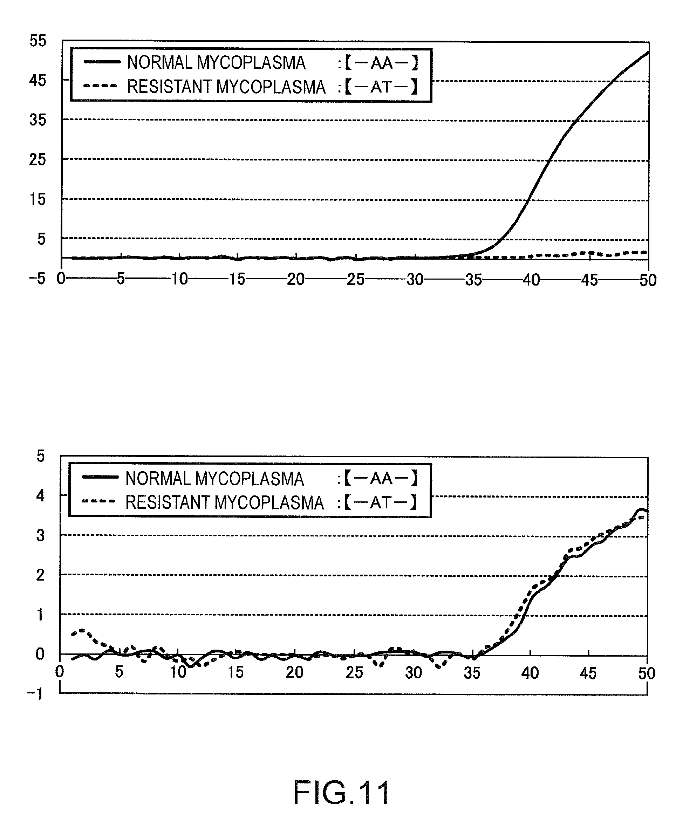

With respect to the above (i) normal type to (vi) resistant type 5, the detection results of fluorescence generated in the above nucleic acid amplification reaction are shown in FIGS. 6 to 11, respectively. The line graph on the upper side shows the results of performing amplification using the normal type probe, and the line graph on the lower side shows the results of performing amplification using the presence/absence probe. The vertical axis represents the fluorescence brightness and the horizontal axis represents the cycle number. The discrimination can be performed as follows: in the case where the fluorescence brightness of both FAM and Cy5.5 increased, the strain is a normal strain, and in the case where the fluorescence brightness of only Cy5.5 increased, the strain is a resistant strain.

As shown in FIGS. 6 to 11, the normal strain and the resistant strain can be discriminated for any mutated form by using only one pair of primers and the following two types of probes: the normal type probe and the presence/absence probe as the probes.

### Example 2

A nucleic acid amplification reaction and fluorescence detection were performed under the same conditions as in Example 1 except that the number of copies of plasmids was changed from 25 copies in Example 1 to 10⁷ copies, and the discrimination between the normal strain and the resistant strain was performed.

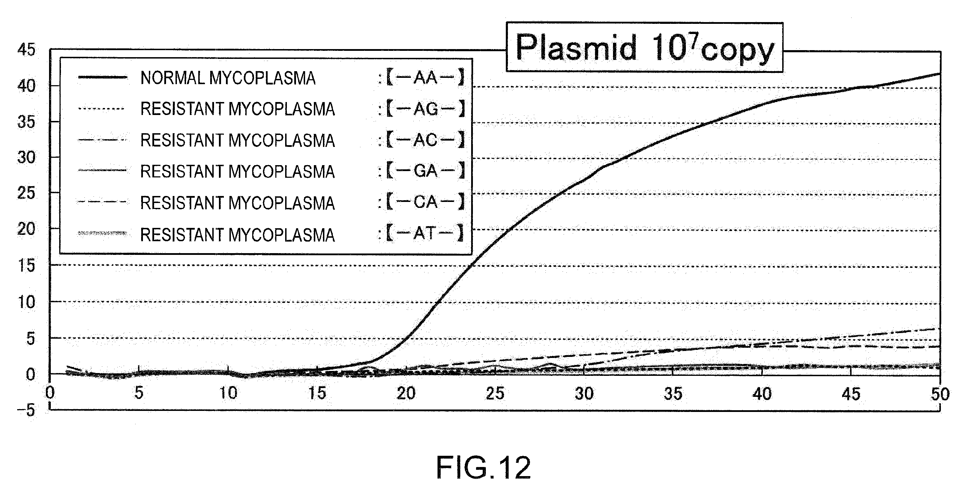

As shown in FIG. 12, even when excess amounts of plasmids were used, the normal strain and the resistant strain could be accurately discriminated. Incidentally, in FIG. 12, the vertical axis represents the fluorescence brightness and the horizontal axis represents the cycle number.

### Example 3

A nucleic acid amplification reaction and fluorescence detection were performed under the same conditions as in Example 1 except that 25 copies of a genomic DNA derived from a normal strain were used in place of the 6 types of the plasmids, and the discrimination between the normal strain and the resistant strain was performed.

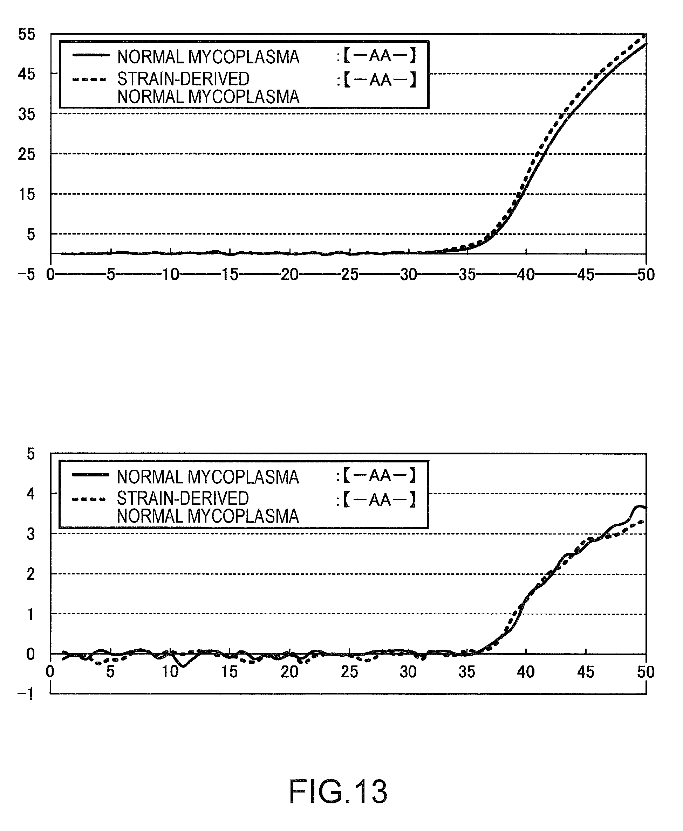

As shown in FIG. 13, even when the genomic DNA was used, the normal strain could be discriminated. Incidentally, in FIG. 13, the vertical axis represents the fluorescence brightness and the horizontal axis represents the cycle number. The line graph on the upper side shows the results of performing amplification using the normal type probe, and the line graph on the lower side shows the results of performing amplification using the presence/absence probe.

### Example 4

A nucleic acid amplification reaction and fluorescence detection were performed under the same conditions as in Example 1 except that a DNA which is a genomic DNA derived from a resistant strain contained in a specimen obtained from a patient suffering from a respiratory disease and has a mutation of "(ii) resistant type 1" in Example 1, 25 copies of a genomic DNA derived from a normal strain, and 25 copies of a normal type plasmid derived from a normal strain were used in place of the 6 types of the plasmids, and the discrimination between the normal strain and the resistant strain was performed.

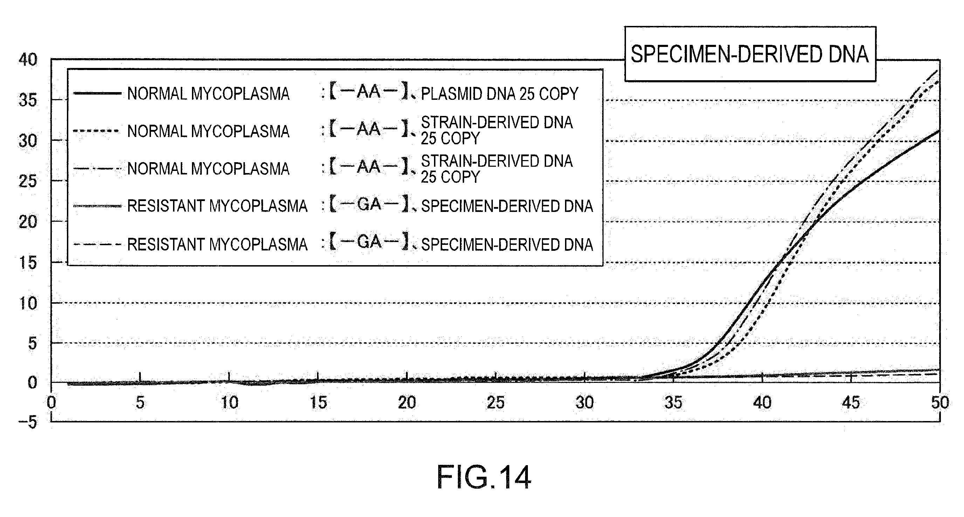

As shown in FIG. 14, even when the genomic DNA was used, the normal strain and the resistant strain could be discriminated. Incidentally, in FIG. 14, the vertical axis represents the fluorescence brightness and the horizontal axis represents the cycle number.

## Claims

1. A method for discriminating the presence or absence of a mutation having a plurality of mutated forms in a DNA, wherein the method comprises performing a thermal cycle for amplifying the DNA in the presence of
one pair of primers which can bind to the DNA,
a first probe which does not bind to the DNA having a mutation, can bind to the DNA having no mutation, and is labeled with a first fluorescent substance, and
a second probe which can bind to both of the DNA having a mutation and the DNA having no mutation and is labeled with a second fluorescent substance which emits a light having a fluorescence wavelength different from that of a light emitted from the first fluorescent substance.

2. The method according to claim 1, wherein the primers bind to the DNA so as to sandwich the DNA regions to which the first and second probes bind.

3. The method according to claim 1 or 2, wherein the strand of the DNA to which the first probe binds is a complementary strand of the DNA to which the second probe binds.

4. The method according to any one of claims 1 to 3,
wherein
the first probe is a TaqMan probe, and
the second probe is a TaqMan probe.

5. The method according to any one of claims 1 to 4,
wherein
the amplification is performed using a nucleic acid amplification reaction apparatus, and
the nucleic acid amplification reaction apparatus includes:
a fitting section capable of fitting a nucleic acid amplification reaction vessel filled with a nucleic acid amplification reaction mixture and a liquid which has a specific gravity smaller than that of the nucleic acid amplification reaction mixture and is immiscible with the nucleic acid amplification reaction mixture;
a first heating section which heats a first region of the nucleic acid amplification reaction vessel;
a second heating section which heats a second region of the nucleic acid amplification reaction vessel; and
a driving mechanism which switches over between a first arrangement in which the first region is located lower than the second region in the direction of the gravitational force and a second arrangement in which the second region is located lower than the first region in the direction of the gravitational force.

6. The method according to any one of claims 1 to 5, wherein the DNA is a DNA derived from Mycoplasma pneumoniae.
